# EUROPEAN PATENT APPLICATION

(11) **EP 4 226 922 A1**
(43) Date of publication of application: **16.08.2023**
(21) Application number: 21891821.7
(22) Date of filing: 08.11.2021
(51) Int. Cl.: A61K 31/407, A61K 9/70, A61K 47/10, A61K 47/18, A61K 47/32, A61K 47/34, A61P 25/18

(54) **METHOD FOR INHIBITING ASENAPINE DEGRADATION**

(30) Priority: 16.11.2020 JP 2020190426
(71) Applicant: Hisamitsu Pharmaceutical Co., Inc., Tosu-shi, Saga 841-0017 (JP)
(72) Inventor: TAKAGI Yuka, Tsukuba-shi, Ibaraki 305-0856 (JP); IWAMOTO Nao, Tsukuba-shi, Ibaraki 305-0856 (JP)
(74) Representative: Grünecker Patent- und Rechtsanwälte PartG mbB
(86) International application number: PCT/JP2021/041002
(87) International publication number: WO 2022/102575

(57) **Abstract**

Disclosed is a method for inhibiting degradation of asenapine or a pharmaceutically acceptable salt thereof in a patch. The patch comprises an adhesive layer on a base, and the adhesive layer contains an adhesive base and asenapine or a pharmaceutically acceptable salt thereof. The method includes incorporating at least one amine compound selected from the group consisting of monoethanolamine, diethanolamine, diisopropanolamine, isopropanolamine, triethanolamine and meglumine in the adhesive layer.

## Description

### Technical Field

The present invention relates to a method for suppress asenapine degradation. In more detail, the present invention relates to a method for suppressing degradation of asenapine or a pharmaceutically acceptable salt thereof in a patch.

### Background Art

Asenapine is a compound known as a medication for treating central nervous system diseases, particularly, schizophrenia. In Japan, as a pharmaceutical containing asenapine, an asenapine maleate sublingual tablet (trade name: SYCREST (registered trademark) sublingual tablet) is in circulation. Ordinarily, sublingual administration is known as a route of administration where the first pass effect is less likely to occur. Therefore, even compounds having relatively low metabolic stability are expected to exhibit a sufficient pharmacological effect when administered sublingually. In recent years, studies have been underway to develop asenapine-containing patches as a new pharmaceutical formulation. For example, Patent Literature 1 discloses a patch containing a low-molecular-weight amine in an adhesive layer, in which a decrease in the adhesive force of the adhesive layer is suppressed even in a case where the adhesive layer absorbs moisture.

### Citation List

### Patent Literature

[Patent Literature 1]
WO 2017/018322

### Summary of Invention

### Technical Problem

The present inventors found that, during the manufacturing and/or storage of patches, there is a possibility that asenapine may be degraded to generate the N-oxide form and tetradehydro form of asenapine. An objective of the present invention is to provide a method for inhibiting degradation of asenapine or a pharmaceutically acceptable salt thereof in a patch.

### Solution to Problem

As a result of intensive studies, the present inventors found that, when a certain amine compound is blended with an adhesive layer, it is possible to suppress asenapine degradation and completed the present invention.

That is, the present invention is a method for inhibiting degradation of asenapine or a pharmaceutically acceptable salt thereof in a patch comprising an adhesive layer on a backing, in which the adhesive layer comprises an adhesive base and asenapine or a pharmaceutically acceptable salt thereof, and degradation of asenapine or the pharmaceutically acceptable salt thereof is suppressed by adding at least one amine compound selected from the group consisting of monoethanolamine, diethanolamine, diisopropanolamine, isopropanolamine, triethanolamine and meglumine in the adhesive layer.

### Advantageous Effects of Invention

According to the present invention, it is possible to provide a patch having excellent stability and comprising asenapine or a pharmaceutically acceptable salt thereof.

### Description of Embodiments

Hereinafter, the present invention will be described in detail by showing an embodiment of the present invention.

An embodiment of the present invention is a method for suppressing degradation of asenapine or a pharmaceutically acceptable salt thereof in a patch comprising an adhesive layer on a backing, in which the adhesive layer comprises an adhesive base and asenapine or a pharmaceutically acceptable salt thereof, and degradation of asenapine or the pharmaceutically acceptable salt thereof is suppressed by adding at least one amine compound selected from the group consisting of monoethanolamine, diethanolamine, diisopropanolamine, isopropanolamine, triethanolamine and meglumine in the adhesive layer.

The backing may be a material capable of maintaining the shape of the patch, particularly, the adhesive layer. Examples of a material of the backing include synthetic resins such as polyethylene, polypropylene, polybutadiene, ethylene-vinyl chloride copolymers, polyvinyl chloride, polyamide such as nylon, polyester, cellulose derivatives and polyurethane. The property of the backing is, for example, a film, a sheet, a sheet-like porous body, a sheet-like foam, a fabric such as woven fabric, knitted fabric or non-woven fabric, a laminate thereof or the like. The thickness of the backing is not particularly limited, but is, normally, preferably approximately 2 µm to 3000 µm.

The adhesive layer is formed of an adhesive composition obtained by mixing an adhesive base, asenapine or a pharmaceutically acceptable salt thereof, at least one amine compound selected from the group consisting of monoethanolamine, diethanolamine, diisopropanolamine, isopropanolamine, triethanolamine and meglumine, and an optional component to be described below. The mass of the adhesive layer per unit area is not particularly limited and can be 30 g/m² to 400 g/m² and may be 40 g/m² to 300 g/m², 50 g/m² to 200 g/m² or 70 g/m² to 120 g/m². When the mass of the adhesive layer per unit area exceeds 400 g/m², the patch is likely to drop when clothes are put on or taken off.

Asenapine is a compound also called (3aRS, 12bRS)-5-chloro-2-methyl-2,3,3a,12b-tetrahydro-1H-dibenzo[2,3:6,7]oxepino[4,5-c]pyrrole and represented by the following formula (1).

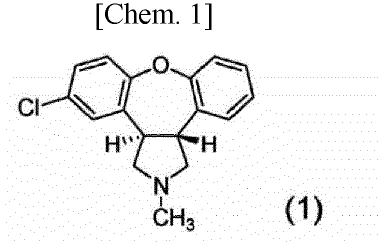

The pharmaceutically acceptable salt of asenapine means, among acid addition salts of asenapine, a salt that can be used as a medicine. Examples of the acid include hydrochloric acid, hydrobromic acid, hydroiodic acid, phosphoric acid, acetic acid, propionic acid, glycolic acid, maleic acid, malonic acid, succinic acid, tartaric acid, citric acid, ascorbic acid, salicylic acid, benzoic acid and the like. For example, asenapine maleate is commercially available as a medication for treating central nervous system diseases.

Asenapine contains a plurality of optical isomers and may be any optical isomer or a mixture of an optical isomer such as racemate. The acid that is added to asenapine is not particularly limited as long as the acid is pharmaceutically acceptable. The acid addition salt of asenapine may be an anhydride or a hydrate.

The content of asenapine or the pharmaceutically acceptable salt thereof can be 1 mass% to 30 mass% and may be 5 mass% to 25 mass%, 7 mass% to 22 mass% or 10 mass% to 20 mass% with respect to the total mass of the adhesive layer.

The adhesive base is a component that imparts adhesiveness to the adhesive layer, and examples thereof include a rubber adhesive base, an acrylic adhesive base, a silicone adhesive base and the like. The adhesive base is preferably one or more selected from the group consisting of a rubber adhesive base, an acrylic adhesive base and a silicone adhesive base. The adhesive base preferably comprises no water (non-aqueous adhesive base). The adhesive base may be any of a rubber adhesive base, an acrylic adhesive base and a silicone adhesive base or may be a combination thereof. The total content of the adhesive base can be 10 mass% to 90 mass% and may be 20 mass% to 90 mass%, 20 mass% to 60 mass% or 20 mass% to 40 mass% with respect to the total mass of the adhesive layer.

Examples of the rubber adhesive base include natural rubber, polyisobutylene, alkyl vinyl ether (co)polymers, polyisoprene, polybutadiene, styrene-butadiene copolymers, styrene-isoprene copolymers, styrene-isoprene-styrene block copolymers (SIS) and the like. As the rubber adhesive base, among these, one base may be used singly or two or more bases may be used in combination. Among these, as the rubber adhesive base according to the present embodiment, at least one selected from the group consisting of styrene-isoprene-styrene block copolymers and polyisobutylene is preferable from the viewpoint of a tendency that a more sufficient adhesive force of the adhesive layer can be exhibited.

Specific examples of the rubber adhesive base include Quintac (registered trademark) 3570C (trade name, manufactured by Zeon Corporation), SIS5002 (trade name, manufactured by JSR Corporation), Oppanol (registered trademark) N50, N80, N100, N150, B11, B12, B50, B80, B100, B120, B150 and B220 (trade names, manufactured by BASF), JSR BUTYL 065, 268 and 365 (trade names, manufactured by JSR Corporation), SIBSTAR (registered trademark) T102 (trade name, manufactured by Kaneka Corporation) and the like.

The content of the rubber adhesive base can be 10 mass% to 90 mass% and may be 20 mass% to 90 mass%, 20 mass% to 60 mass% or 20 mass% to 40 mass% with respect to the total mass of the adhesive layer.

The acrylic adhesive base is a component that imparts adhesiveness to the adhesive layer and is, for example, one or more (co)polymers of a alkyl (meth)acrylate. Examples of the alkyl (meth)acrylate include butyl (meth)acrylate, isobutyl (meth)acrylate, hexyl (meth)acrylate, octyl (meth)acrylate, 2-ethylhexyl (meth)acrylate, decyl (meth)acrylate and the like. In the present specification, the term "(meth)acrylic acid" means any one or both of acrylic acid and methacrylic acid, and similar expressions are also defined in the same manner.

The acrylic adhesive base may be a copolymer formed of the alkyl (meth)acrylate (main monomer) and a comonomer. Examples of the main monomer include methyl (meth)acrylate, ethyl (meth)acrylate, butyl (meth)acrylate, hexyl (meth)acrylate, heptyl (meth)acrylate, octyl (meth)acrylate, 2-ethylhexyl (meth)acrylate and the like, and, among these, one monomer may be used singly or two or more monomers may be used in combination. The comonomer may be a component that can be copolymerized with the alkyl (meth)acrylate. Examples of the comonomer include hydroxyalkyl (meth)acrylate, ethylene, propylene, styrene, vinyl acetate, N-vinylpyrrolidone, (meth)acrylic acid, (meth)acryl amide and the like. The comonomer may be a single comonomer or a combination of two or more comonomers.

Specific examples of the acrylic adhesive base include acrylic acid/octyl acrylate copolymers, 2-ethylhexyl acrylate/vinylpyrrolidone copolymer solutions, acrylate/vinyl acetate copolymers, 2-ethylhexyl acrylate/2-ethylhexyl methacrylate/dodecyl methacrylate copolymers, methyl acrylate/2-ethylhexyl acrylate copolymer resin emulsions, acrylic polymers that are contained in acrylic resin alkanolamine liquids and the like. Specific examples of such an acrylic adhesive include the DURO-TAK series (manufactured by Henkel AG & Co. KGaA) such as DURO-TAK (registered trademark) 387-2510, DURO-TAK (registered trademark) 87-2510, DURO-TAK (registered trademark) 387-2287, DURO-TAK (registered trademark) 87-2287, DURO-TAK (registered trademark) 87-4287, DURO-TAK (registered trademark) 387-2516, DURO-TAK (registered trademark) 87-2516, DURO-TAK (registered trademark) 87-2074, DURO-TAK (registered trademark) 87-900A, DURO-TAK (registered trademark) 87-901A, DURO-TAK (registered trademark) 87-9301 and DURO-TAK (registered trademark) 87-4098; the GELVA series (manufactured by Henkel AG & Co. KGaA) such as GELVA (registered trademark) GMS788, GELVA (registered trademark) GMS3083 and GELVA (registered trademark) GMS 3253; the MAS series (manufactured by CosMed Pharmaceutical Co., Ltd.) such as MAS811 (trade name) and MAS683 (trade name); the Eudragit (registered trademark) series (manufactured by EVONIK Industries AG), the NIKASOL (registered trademark) series (manufactured by Nippon Carbide Industries Co., Ltd.) and the ULTRAZORU (registered trademark) series (manufactured by Aica Kogyo Co., Ltd.).

The content of the acrylic adhesive base can be 10 mass% to 90 mass% and may be 20 mass% to 90 mass%, 20 mass% to 60 mass% or 20 mass% to 40 mass% with respect to the total mass of the adhesive layer.

The silicone adhesive base is a compound having an organopolysiloxane skeleton. Examples of the silicone adhesive base include dimethylpolysiloxane, polymethylvinylsiloxane and polymethylphenylsiloxane. Examples of specific silicone adhesive bases include the MD series (manufactured by DuPont Toray Specialty Materials K.K.) such as MD7-4502 Silicone Adhesive and MD7-4602 Silicone Adhesive; the BIO-PSA series (manufactured by DuPont Toray Specialty Materials K.K.) such as Liveo (registered trademark) BIO-PSA 7-4301 Silicone Adhesive, Liveo (registered trademark) BIO-PSA 7-4302 Silicone Adhesive, Liveo (registered trademark) BIO-PSA 7-4201 Silicone Adhesive, Liveo (registered trademark) BIO-PSA 7-4202 Silicone Adhesive, Liveo (registered trademark) BIO-PSA 7-4101 Silicone Adhesive, Liveo (registered trademark) BIO-PSA 7-4102 Silicone Adhesive, Liveo (registered trademark) BIO-PSA 7-4601 Silicone Adhesive, Liveo (registered trademark) BIO-PSA 7-4602 Silicone Adhesive, Liveo (registered trademark) BIO-PSA 7-4501 Silicone Adhesive, Liveo (registered trademark) BIO-PSA 7-4502 Silicone Adhesive, Liveo (registered trademark) BIO-PSA 7-4401 Silicone Adhesive and Liveo (registered trademark) BIO-PSA 7-4402 Silicone Adhesive, Dow Coming (registered trademark) 7-9800A, Dow Coming (registered trademark) 7-9800B, Dow Coming (registered trademark) 7-9700A and Dow Coming (registered trademark) 7-9700B.

The content of the silicone adhesive base can be 10 mass% to 90 mass% and may be 20 mass% to 90 mass%, 20 mass% to 60 mass% or 20 mass% to 40 mass% with respect to the total mass of the adhesive layer.

Adding at least one amine compound selected from the group consisting of monoethanolamine, diethanolamine, diisopropanolamine, isopropanolamine, triethanolamine and meglumine in the adhesive layer, suppresses the generation of the N-oxide form and tetradehydro form of asenapine during the manufacturing and/or storage of the patch. The amine compound is preferably at least one selected from the group consisting of diethanolamine, diisopropanolamine, triethanolamine and meglumine and more preferably at least one selected from the group consisting of diethanolamine, diisopropanolamine and triethanolamine.

The content of the amine compound can be 0.01 mass% to 1 mass% and may be 0.05 mass% to 1 mass%, 0.05 mass% to 0.5 mass% or 0.1 mass% to 0.5 mass% with respect to the total mass of the adhesive layer.

The adhesive layer may, optionally, further comprise other additives. Examples of the other additives include a tackifying resin, a plasticizer, an absorption enhancer, a solubilizer, other auxiliary stabilizers, a filler, a fragrance and the like.

The tackifying resin is a component that adjusts the adhesiveness of the adhesive layer. Examples of the tackifying resin include alicyclic saturated hydrocarbon resins; rosin derivatives such as rosin, glycerol ester of rosin, hydrogenated rosin, glycerol ester of hydrogenated rosin, pentaerythritol ester of rosin and maleated rosin; terpene tackifying resins; petroleum tackifying resins and the like. The tackifying resin may be used singly or two or more tackifying resins may be used in combination. In a case where the adhesive layer comprises the tackifying resin, the content of the tackifying resin can be 20 mass% to 80 mass% and may be 30 mass% to 70 mass% with respect to the total mass of the adhesive layer.

Examples of the plasticizer include paraffin oils (liquid paraffin and the like), squalane, squalene, vegetable oils (olive oil, camellia oil, castor oil, tall oil, peanut oil, spearmint oil, eucalyptus oil, jojoba oil, white camphor oil, sunflower oil, orange oil and the like), oils and fats (dibutyl phthalate, dioctyl phthalate and the like) and liquid rubber (liquid polybutene, liquid isoprene rubber and the like). A preferable plasticizer is liquid paraffin or liquid polybutene. In a case where the adhesive layer comprises the plasticizer, the content of the plasticizer is, for example, 3 mass% to 50 mass%, 5 mass% to 30 mass% or 7 mass% to 20 mass% with respect to the total mass of the adhesive layer.

The absorption enhancer may be a compound that is conventionally known to have a transdermal absorption promoting action. Examples of the absorption enhancer include organic acids and salts thereof (for example, aliphatic carboxylic acids having 6 to 20 carbon atoms (hereinafter, also referred to as "fatty acids") and salts thereof, cinnamic acid and salts thereof), organic acid esters (for example, fatty acid esters and cinnamic esters), organic acid amides (for example, fatty acid amides), fatty alcohols, polyhydric alcohols, ethers (for example, fatty ethers and polyoxyethylene alkyl ethers) and the like. These absorption enhancers may have an unsaturated bond or may be a cyclic, linear or branched chemical structure. In addition, the absorption enhancer may be a monoterpene compound, a sesquiterpene compound, and a vegetable oil (for example, olive oil). These absorption enhancers may be used singly or two or more absorption enhancers may be used in combination.

Examples of such organic acids include aliphatic (mono-, di- or tri)carboxylic acids (for example, acetic acid, propionic acid, citric acid (including anhydrous citric acid), isobutyric acid, caproic acid, caprylic acid, fatty acid, lactic acid, maleic acid, pyruvic acid, oxalic acid, succinic acid, tartaric acid and the like), aromatic carboxylic acids (for example, phthalic acid, salicylic acid, benzoic acid, acetylsalicylic acid and the like), cinnamic acid, alkanesulfonic acids (for example, methanesulfonic acid, ethanesulfonic acid, propanesulfonic acid and butanesulfonic acid), alkylsulfonic acid derivatives (for example, polyoxyethylene alkyl ether sulfonic acid, N-2-hydroxyethylpiperazine-N'-2-ethanesulfonic acid) and cholic acid derivatives (for example, dehydrocholic acid and the like). These organic acids may be alkali metal salts such as sodium salts. Among them, aliphatic carboxylic acids, aromatic carboxylic acids or salts thereof are preferable, and acetic acid, sodium acetate or citric acid is particularly preferable. Examples of the fatty acid include lauric acid, myristic acid, palmitic acid, stearic acid, isostearic acid, oleic acid, linoleic acid and linolenic acid.

Examples of the organic acid esters include ethyl acetate, propyl acetate, cetyl lactate, lauryl lactate, methyl salicylate, ethylene glycol salicylate, methyl cinnamate and fatty acid esters. Examples of the fatty acid esters include methyl laurate, hexyl laurate, isopropyl myristate, myristyl myristate, octyldodecyl myristate, isopropyl palmitate and cetyl palmitate. The fatty acid esters may be glycerin fatty acid ester, propylene glycol fatty acid ester, sorbitan fatty acid ester, polyethylene glycol sorbitan fatty acid ester, polyethylene glycol fatty acid ester, sucrose fatty acid ester or polyoxyethylene hydrogenated castor oil. Specific examples of the fatty acid esters include glycerin monocaprylate, glycerin monocaprate, glycerin monolaurate, glycerin monooleate, sorbitan monolaurate, sucrose monolaurate, polysorbate 20, propylene glycol monolaurate, polyethylene glycol monolaurate, polyethylene glycol monostearate, Span40, Span60, Span80, Span120 (trade names, manufactured by Croda Japan K.K.), Tween20, Tween21, Tween40, Tween60, Tween80 and NIKKOL HCO-60 (trade names, manufactured by Nikko Chemicals Co., Ltd.).

Examples of the organic acid amides include fatty acid amides (for example, lauric acid diethanolamide), hexahydro-1-dodecyl-2H-azepin-2-one (also called azone) and derivatives thereof and pyrothiodecane.

The fatty alcohols mean fatty alcohols having 6 to 20 carbon atoms. Examples of the fatty alcohols include lauryl alcohol, myristyl alcohol, oleyl alcohol, isostearyl alcohol and cetyl alcohol. As the polyhydric alcohols, for example, propylene glycol is an exemplary example.

The fatty ethers mean ethers having an aliphatic group having 6 to 20 carbon atoms (for example, an alkyl group and an alkenyl group). As the polyoxyethylene alkyl ethers, for example, polyoxyethylene lauryl ether is an exemplary example.

Examples of the monoterpene compound include geraniol, thymol, terpineol, 1-menthol, borneol, d-limonene, isoborneol, nerol and dl-camphor. As the monoterpene compound, peppermint oil may be used.

As the absorption enhancer, a fatty acid (particularly oleic acid), isopropyl palmitate, oleyl alcohol, lauryl alcohol, isostearyl alcohol, lauric acid diethanolamide, glycerin monocaprylate, glycerin monocaprate, glycerin monooleate, sorbitan monolaurate, propylene glycol monolaurate, polyoxyethylene lauryl ether or pyrothiodecane is more preferable.

In a case where the adhesive layer comprises the absorption enhancer, the content of the absorption enhancer can be 2 mass% to 40 mass% with respect to the total mass of the adhesive layer.

The solubilizer is a component that makes it easy for asenapine or the pharmaceutically acceptable salt thereof to be dissolved in the adhesive composition. Examples of the solubilizer include fatty acids (for example, capric acid, oleic acid and linoleic acid), fatty acid alkyl esters (for example, isopropyl myristate and isopropyl palmitate), fatty acid polyhydric alcohol esters (for example, propylene glycol monolaurate, glyceryl monolaurate, glyceryl monooleate and sorbitan monolaurate), fatty acid amides (for example, lauric acid diethanolamide), fatty alcohols (for example, octyldodecanol, isostearyl alcohol and oleyl alcohol), polyhydric alcohols (for example, propylene glycol, dipropylene glycol and polyethylene glycol), pyrrolidone derivatives (for example, N-methyl-2-pyrrolidone), organic acids or salts thereof (for example, acetic acid, lactic acid, sodium acetate and sodium lactate) and sodium hydroxide. In a case where the adhesive layer comprises the solubilizer, the content of the solubilizer can be 2 mass% to 40 mass% with respect to the total mass of the adhesive layer.

The other auxiliary stabilizers may be an additive capable of suppressing the generation of a free radical that is generated by the action of light rays such as ultraviolet rays, heat or active species and the progress of a chain reaction thereof. When a stabilizer is optionally comprised, it is possible to further improve the stability of asenapine during the manufacturing of the patch. Examples of the stabilizer include tocopherol and ester derivatives thereof, ascorbic acid and ester derivatives thereof, 2,6-dibutylhydroxytoluene (BHT), 2,6-dibutylhydroxyanisole (BHA), 2-mercaptobenzimidazole and the like. The stabilizer may be used singly or two or more stabilizers may be used in combination. As the stabilizer, dibutylhydroxytoluene is preferable because asenapine is further stabilized. In a case where the adhesive layer comprises the stabilizer, the content of the stabilizer can be 0.05 mass% to 3 mass% and may be 0.05 mass% to 1 mass%, 0.05 mass% to 0.25 mass% or 0.1 mass% to 0.25 mass% with respect to the total mass of the adhesive layer. When the content of the stabilizer is 0.05 mass% to 3 mass%, there is a tendency that the stability of each component in the patch is excellent.

Examples of the filler include the powders of a metal compound (aluminum oxide, aluminum hydroxide, zinc oxide, titanium oxide, calcium carbonate or the like), ceramic (talc, clay, kaolin, silica, hydroxyapatite, synthetic aluminum silicate, magnesium aluminometasilicate or the like) or an organic compound (cellulose powder, stearate or the like) or short fibers of a resin containing these. In a case where the adhesive layer contains the filler, the content of the filler can be 0.1 mass% to 20 mass% with respect to the total mass of the adhesive layer.

The patch may further comprise a release liner. The release liner is laminated on the surface of the adhesive layer opposite to the backing. When the release liner is provided, there is a tendency that it is possible to reduce attachment of rubber or the like to the adhesive layer during storage. On the surface of the release liner that comes into contact with the adhesive layer, it is preferable to perform a release treatment with silicone, fluorinated polyolefin or the like.

A material of the release liner is not particularly limited, and it is possible to use liners that are ordinarily known to persons skilled in the art. Examples of the release liner include films of a polyester such as polyethylene terephthalate or polyethylene naphthalate; a polyolefin such as polyethylene or polypropylene; polyvinyl chloride, polyvinylidene chloride, nylon, aluminum or the like. The release liner may be a laminate film between high-quality paper and polyolefin. As the material of the release liner, a polypropylene or polyethylene terephthalate film is preferable.

The patch can be manufactured by, for example, the following method, but the manufacturing method is not limited thereto, and it is possible to use a well-known method. First, the individual components of the adhesive layer are mixed together in predetermined proportions to obtain a homogeneous dissolved matter (adhesive composition). Next, an adhesive layer is formed by spreading the adhesive composition in a predetermined thickness on a releasable film (release liner). Furthermore, a backing is bonded by pressure to the adhesive layer so that the adhesive layer is sandwiched between the release liner and the backing. Finally, the laminate is cut to a desirable shape and dimensions, whereby a patch can be obtained. In this case, the release liner is removed at the time of applying the patch. The shape and dimensions of the patch may be, for example, a rectangular shape that is 3 to 14 cm long in the short side and 7 to 20 cm long in the long side or a round shape having a diameter of 1 to 10 cm.

### [Examples]

### Test Example 1: Stability evaluation of patch comprising various amine compounds

### Preparation of patch

Individual components were mixed together in accordance with Table 1 to Table 3 below to obtain adhesive compositions. Each of the obtained adhesive compositions was spread on a release liner (a polyethylene terephthalate film on which a release treatment had been performed) so that the mass per unit area reached 100 g/m², and a solvent was dried and removed to form an adhesive layer. A backing layer (polyethylene terephthalate film) was laminated on the opposite surface of the obtained adhesive layer, thereby obtaining a patch in which the backing layer, the adhesive layer and the release liner were laminated in this order.

### Content test

For the patch that had just been manufactured by the above-described manufacturing method and the patch that had been stored in a packaging bag at 60°C for one month, the amounts of the N-oxide form and tetradehydro form of asenapine generated were quantified by HPLC.

Specifically, the adhesive layer of the patch was taken out and immersed in 5 mL of tetrahydrofuran (high-performance liquid chromatography grade) to extract an organic matter, 45 mL of a diluted solution (0.1% phosphoric acid aqueous solution/methanol = 50/50 (v/v)) was added thereto to adjust the total amount to 50 mL, an insoluble matter was filtered, and then a chromatograph on which the peaks of asenapine and the N-oxide form and tetradehydro form thereof were separated was obtained by high-performance liquid chromatography under the following analysis conditions. The contents of the N-oxide form and the tetradehydro form were calculated from the values of the areas under the curves of the peaks corresponding to the N-oxide form and the tetradehydro form with an assumption that the theoretical amount of asenapine was regarded as 100. The relative retention time (RRT) of the N-oxide form relative to asenapine was 0.24, and RRT of the tetradehydro form relative to asenapine was 1.10.

### <Analysis conditions>

Column: CAPCELLPACKC18 MGII 5 µm (4.6 mm I.D × 150 mm)
Mobile phase: Methanol/phosphoric acid buffer solution (pH 6.8) = 70/30
Measurement wavelength: 230 nm
Flow rate: 1.0 mL/min
Sample injection amount: 15 µL
Column temperature: 50°C

**[Table 1]**

| | Comparative Example | | | | | | |
|---|---|---|---|---|---|---|---|
| | 1 | 2 | 3 | 4 | 5 | 6 | 7 |
| Asenapine free base | 14 | 14 | 14 | 14 | 14 | 14 | 14 |
| SIS | 26.88 | 26.87 | 26.56 | 26.86 | 26.56 | 26.87 | 26.56 |
| Aliphatic saturated hydrocarbon resin | 59.12 | 59.11 | 58.44 | 59.09 | 58.44 | 59.11 | 58.44 |
| Ascorbyl palmitate | - | 0.02 | 1 | - | - | - | - |
| Propyl gallate | - | - | - | 0.05 | 1 | - | - |
| Tocopherol | - | - | - | - | - | 0.02 | 1 |
| Total (%) | 100 | 100 | 100 | 100 | 100 | 100 | 100 |
| N-oxide form (immediately after manufacture) | 0 | 0 | 0.47 | 0 | 0.07 | 0 | 0.04 |
| N-oxide form (after storing at 60°C for one month) | 0.47 | 0.4 | 0.51 | 0.53 | 2.42 | 0.4 | 1.96 |
| Tetradehydro form (immediately after manufacture) | 0.1 | 0.1 | 0.08 | 0.06 | 0.07 | 0.25 | 0.08 |
| Tetradehydro form (after storing at 60°C for one month) | 0.51 | 0.49 | 0.51 | 0.49 | 1.2 | 0.6 | 1.74 |

**[Table 2]**

| | Example | | | | | | | | |
|---|---|---|---|---|---|---|---|---|---|
| | 1 | 2 | 3 | 4 | 5 | 6 | 7 | 8 | 9 |
| Asenapine free base | 14 | 14 | 14 | 14 | 14 | 14 | 14 | 14 | 14 |
| SIS | 26.84 | 26.78 | 26.72 | 26.72 | 26.84 | 26.78 | 26.84 | 26.78 | 26.72 |
| Aliphatic saturated hydrocarbon resin | 59.06 | 58.92 | 58.78 | 58.78 | 59.06 | 58.92 | 59.06 | 58.92 | 58.78 |
| Diethanolamine | 0.1 | 0.3 | 0.5 | - | - | - | - | - | - |
| Diisopropanolamine | - | - | - | 0.5 | - | - | - | - | - |
| Triethanolamine | - | - | - | - | 0.1 | 0.3 | - | - | - |
| Meglumine | - | - | - | - | - | - | 0.1 | 0.3 | 0.5 |
| Total (%) | 100 | 100 | 100 | 100 | 100 | 100 | 100 | 100 | 100 |
| N-oxide form (immediately after manufacture) | 0 | 0 | 0 | 0 | 0 | 0 | 0 | 0 | 0 |
| N-oxide form (after storing at 60°C for one month) | 0.12 | 0.05 | 0.04 | 0.13 | 0.11 | 0.08 | 0.24 | 0.1 | 0.04 |
| Tetradehydro form (immediately after manufacture) | 0 | 0 | 0.03 | 0.03 | 0 | 0.02 | 0.02 | 0.02 | 0.02 |
| Tetradehydro form (after storing at 60°C for one month) | 0.18 | 0.18 | 0.12 | 0.2 | 0.19 | 0.2 | 0.25 | 0.17 | 0.24 |

**[Table 3]**

| | Example | | | | | |
|---|---|---|---|---|---|---|
| | 10 | 11 | 12 | 13 | 14 | 15 |
| Asenapine free base | 14 | 14 | 14 | 14 | 14 | 14 |
| SIS | 26.7 | 26.75 | 26.77 | 26.77 | 26.81 | 26.83 |
| Aliphatic saturated hydrocarbon resin | 58.75 | 58.85 | 58.88 | 58.88 | 58.99 | 59.02 |
| Triethanolamine | 0.3 | 0.3 | 0.3 | 0.1 | 0.1 | 0.1 |
| BHT | 0.25 | 0.1 | 0.05 | 0.25 | 0.1 | 0.05 |
| Total (%) | 100 | 100 | 100 | 100 | 100 | 100 |
| N-oxide form (immediately after manufacture) | 0 | 0 | 0 | 0 | 0 | 0 |
| N-oxide form (after storing at 60°C for one month) | 0.07 | 0.07 | 0.07 | 0.09 | 0.07 | 0.08 |
| Tetradehydro form (immediately after manufacture) | 0 | 0.02 | 0.02 | 0.02 | 0 | 0.02 |
| Tetradehydro form (after storing at 60°C for one month) | 0.12 | 0.13 | 0.14 | 0.1 | 0.14 | 0.14 |

It was confirmed that, in the patches comprising diethanolamine, diisopropanolamine, triethanolamine or meglumine, the generation of the N-oxide form and tetradehydro form of asenapine was sufficiently suppressed. In addition, it was confirmed that, in the patches comprising triethanolamine and BHT, the generation of degraded products was further suppressed.

### Test Example 2: Stability evaluation of patch comprising amine compound

Individual components were mixed together in according with Table 4 and Table 5 below to obtain adhesive compositions. Patches were obtained in the same manner as in Test Example 1. The amounts of the N-oxide forms and tetradehydro forms of asenapine generated in the patches that had just been manufactured and the patches that had been stored in a packaging bag at 60°C for one month were quantified by HPLC in the same manner as in Test Example 1.

**[Table 4]**

| | Example | | | |
|---|---|---|---|---|
| | 16 | 17 | 18 | 19 |
| Asenapine free base | 14 | 14 | 14 | - |
| Asenapine maleate | - | - | - | 20 |
| SIS | 26.86 | 26.56 | 59.85 | 23.6 |
| Polyisobutylene | - | - | 25.65 | - |
| Aliphatic saturated hydrocarbon resin | 59.09 | 58.44 | - | 51.92 |
| Triethanolamine | 0.05 | 1 | 0.5 | 0.5 |
| Sodium hydroxide | - | - | - | 3.98 |
| Total | 100 | 100 | 100 | 100 |
| N-oxide form (immediately after manufacture) | 0 | 0 | 0.05 | 0 |
| N-oxide form (after storing at 60°C for one month) | 0.27 | 0.17 | 0.23 | 0.16 |
| Tetradehydro form (immediately after manufacture) | 0 | 0 | 0 | 0 |
| Tetradehydro form (after storing at 60°C for one month) | 0.29 | 0.25 | 0.40 | 0.19 |

**[Table 5]**

| | Comparative Example 8 | Example 20 | Comparative Example 9 | Example 21 |
|---|---|---|---|---|
| Asenapine free base | 14 | 14 | 14 | 14 |
| Acrylic pressure-sensitive adhesive base DURO-TAK^{™} 87-900A | 86 | 85.5 | - | - |
| Silicone-based pressure-sensitive adhesive base Liveo^{™} BIO-PSA 7-4202 | - | - | 86 | 85.5 |
| Triethanolamine | - | 0.5 | - | 0.5 |
| Total | 100 | 100 | 100 | 100 |
| N-oxide form (immediately after manufacture) | 0.59 | 0.53 | 1.25 | 0.72 |
| N-oxide form (after storing at 60°C for one month) | 4.4 | 1.6 | 5.7 | 0.4 |
| Tetradehydro form (immediately after manufacture) | 0.23 | 0.21 | 0.62 | 0.44 |
| Tetradehydro form (after storing at 60°C for one month) | 1.39 | 0.52 | 2.49 | 1.93 |

It was confirmed that, in the patches comprising triethanolamine, the generation of the N-oxide form and tetradehydro form of asenapine was sufficiently suppressed.

## Claims

1. A method for suppressing degradation of asenapine or a pharmaceutically acceptable salt thereof in a patch,
wherein the patch comprises an adhesive layer on a backing, the adhesive layer comprises an adhesive base and asenapine or a pharmaceutically acceptable salt thereof, and
comprising adding at least one amine compound selected from the group consisting of monoethanolamine, diethanolamine, diisopropanolamine, isopropanolamine, triethanolamine and meglumine in the adhesive layer.

2. The method according to Claim 1,
wherein the adhesive base is at least one selected from the group consisting of a rubber adhesive base, an acrylic adhesive base and a silicone adhesive base.

3. The method according to Claim 1 or 2,
wherein a content of the amine compound is 0.01 mass% to 1 mass% based with respect to a total mass of the adhesive layer.

4. The method according to any one of Claims 1 to 3,
comprising further adding dibutylhydroxytoluene in the adhesive layer.
